# FASCICULE DE BREVET EUROPEEN

(11) **EP 3 272 381 B1**
(45) Date de publication et mention de la délivrance du brevet: **13.02.2019**
(21) Numéro de dépôt: 17181015.3
(22) Date de dépôt: 12.07.2017
(51) Int. Cl.: A61M 15/00, A61M 15/08

(54) **DISPOSITIF INHALATEUR ET SYSTEME DE TRAITEMENT**
INHALATIONSVORRICHTUNG UND BEHANDLUNGSSYSTEM
INHALER DEVICE AND TREATMENT SYSTEM

(30) Priorité: 22.07.2016 FR 1657001
(43) Date de publication de la demande: 24.01.2018
(73) Titulaire: LA DIFFUSION TECHNIQUE FRANCAISE, 42000 Saint Etienne (FR); Université François-Rabelais de Tours, 37000 Tours (FR)
(72) Inventeur: VECELLIO-NONE, Laurent, 37170 CHAMBRAY LES TOURS (FR)
(74) Mandataire: Cabinet Laurent & Charras

(56) Documents cités:
- EP-A1- 1 674 122
- WO-A1-01/35856
- WO-A1-2004/091704
- WO-A1-2010/149280
- WO-A1-2013/098714
- WO-A2-01/78818
- WO-A2-02/05630
- WO-A2-2006/124954
- FR-A1- 2 948 288
- GB-A- 975 754

## Description

La présente invention concerne un dispositif inhalateur, notamment pour le traitement des pathologies nasales. L'invention concerne également un système de traitement, comprenant un tel dispositif inhalateur et un générateur d'aérosol doseur.

Le domaine de l'invention est celui du traitement des pathologies nasales, notamment de la rhinosinusite, et en particulier la rhinosinusite chronique (ci-après « RSC »).

En pratique, la RSC est une maladie inflammatoire de la muqueuse nasale et des cavités sinusiennes paranasales. Elle se manifeste par des symptômes comme l'obstruction nasale, l'écoulement antérieur et/ou postérieur, des douleurs ou pressions faciales et des troubles olfactifs, pendant plus de 12 semaines.

L'administration topique de corticoïdes est recommandée, selon un grade A, en association ou non avec une antibiothérapie orale. Cette administration topique est actuellement réalisée à l'aide de pompes nasales (sprays) produisant des particules d'une taille de l'ordre de 50 µm. L'utilisation de ce mode d'administration génère un dépôt proximal dans les cavités nasales et ne permet pas d'atteindre des zones davantage inflammées.

L'étude récente publiée par Reychler et al (Effect of three-drug delivery modalities on olfactory function in chronic sinusitis. Reychler G, Colbrant C, Huart C, Le Guellec S, Vecellio L, Liistro G, Rombaux P. Laryngoscope. 2015 Mar;125(3):549-55. doi: 10.1002/lary.24937*. Epub 2014 Sep* 16.) a démontré que l'utilisation de particules plus fines (environ 2 µm) produites par nébulisation permettait de cibler la périphérie des cavités nasales inflammées, contrairement aux sprays. Les tests d'olfactions associés à cette étude ont mis en évidence la supériorité significative du traitement par nébulisation de corticoïdes en comparaison des sprays de corticoïdes, pour une même dose. Cette étude démontre qu'il est donc important de cibler les zones inflammées en périphérie pour augmenter l'efficacité du traitement.

Les pompes nasales sont des dispositifs produisant des sprays de particules de l'ordre de 50 µm à l'aide d'un injecteur. Le médicament est directement conditionné sous forme liquide dans le dispositif. L'administration du traitement ne dure que quelques secondes. Ces dispositifs sont par exemple décrits dans des brevets de la société Aptar (US 4830284 A). Ces dispositifs ne sont pas totalement efficaces car le dépôt du médicament n'atteint pas les zones périphériques des cavités nasales, dues à leurs fortes vitesses d'injection et leurs larges tailles de particules. De plus, le spray liquide déposé dans les cavités nasales est associé à un écoulement du liquide médicamenteux dans la gorge provocant un gout désagréable pour le patient et limitant le temps de résidence du médicament dans les cavités nasales.

Les nébuliseurs sont des dispositifs demandant une préparation préalable, consistant à introduire le médicament dans le dispositif, avant son administration au patient. Le nébuliseur doit ensuite être nettoyé après la séance. Les nébuliseurs produisent des particules d'environ 2 à 5µm et nécessitent un temps d'inhalation d'environ 10 minutes. Ces dispositifs sont efficaces pour cibler les zones périphériques des cavités nasales, mais posent le problème de la compliance des patients au traitement compte tenu du temps de l'administration du médicament.

Récemment, la société Teva a développé un dispositif nasal (US 20120085345) à partir du principe d'aérosol doseur avec gaz pressurisé. Le dispositif est composé d'une cartouche et d'un embout nasal. Ce dispositif possède l'avantage d'être simple, rapide (quelques secondes de temps d'administration du traitement) et de produire une certaine quantité de particules fines, mais ne résout pas totalement le problème posé. En effet, les particules produites à l'extrémité de l'embout nasal ont une forte vitesse d'injection et n'ont pas totalement le temps de s'évaporer : elles sont donc associées à une forte impaction dans les premiers centimètres des fosses nasales (Nasal Déposition of HFA-Beclomethasone, Aqueous Fluticasone Propionate and Aqueous Mometasone Furoate in Allergic Rhinitis Patients No Access ; Chet L. Leach, Philip J. Kuehl, Ramesh Chand, Jacob D. McDonald ; Journal of Aerosol Medicine and Pulmonary Drug Delivery. Oct 2015, 28(5): 334-340).

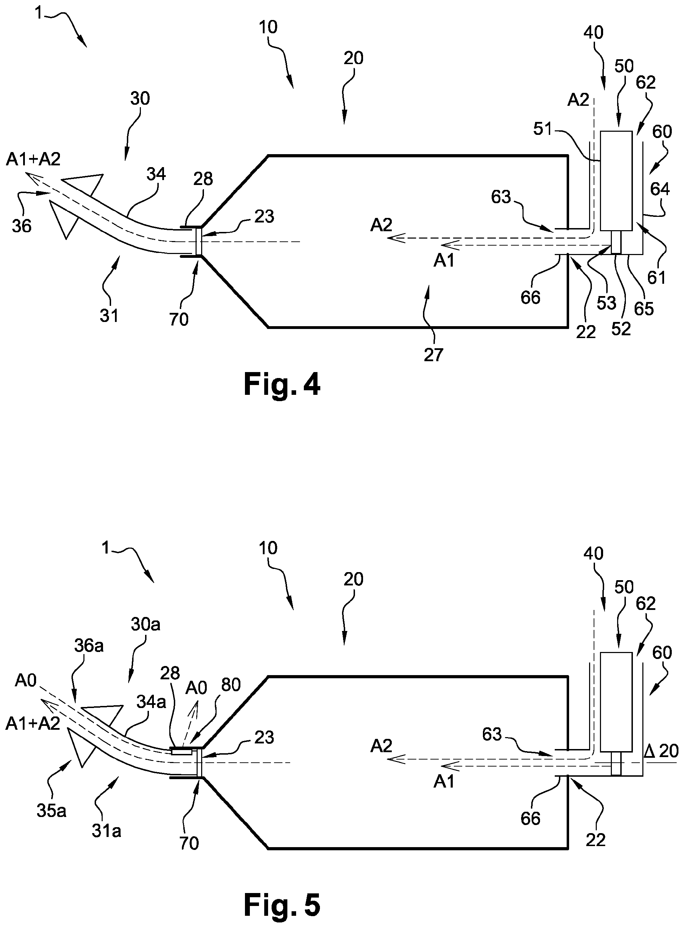

WO 2013/098714 A1 décrit un dispositif inhalateur (par exemple figure 4C), destiné à recevoir un générateur d'aérosol, pour le traitement des pathologies nasales chez un patient. Cependant, ce dispositif ne présente pas d'angle d'inclinaison entre l'embout nasal et la chambre d'inhalation (voir figures 4A à 4D). Ainsi, ce dispositif ne permet pas de cibler les zones anatomiques d'intérêt, en particulier de favoriser la pénétration d'aérosol dans les zones supérieures des cavités nasales.

D'autres dispositifs inhalateurs connus de l'art antérieur sont décrits par exemple dans les documents WO 2010/149280 A1, WO 02/05630 A2, WO 01/35856 A1, WO 2006/124954 A2 et WO 01/78818 A2.

Le but de la présente invention est de proposer un dispositif visant à remédier aux inconvénients ci-dessus.

A cet effet, l'invention a pour objet un dispositif inhalateur, destiné à recevoir un générateur d'aérosol doseur, pour le traitement des pathologies nasales chez un patient, le dispositif inhalateur étant configuré pour être utilisé horizontalement et comprenant une chambre d'inhalation présentant un axe longitudinal horizontal, une interface nasale du type embout nasal comportant au moins une ouverture de sortie, une valve inspiratoire unidirectionnelle, et une valve expiratoire disposée entre la valve inspiratoire et l'au moins une ouverture de sortie de l'interface nasale ; dans lequel la chambre d'inhalation comporte un corps allongé suivant l'axe longitudinal horizontal et délimitant un volume intérieur pour l'évaporation et le freinage d'un aérosol délivré par le générateur d'aérosol doseur, une ouverture aval connectée à l'interface nasale, et une ouverture amont destinée à faire pénétrer de l'air extérieur dans le volume intérieur de la chambre d'inhalation pour assurer le transport efficace de l'aérosol vers l'ouverture aval ; dans lequel la valve inspiratoire est disposée au niveau de l'ouverture aval ou de l'ouverture amont de la chambre d'inhalation ; et dans lequel l'interface nasale est configurée pour guider l'aérosol et l'air extérieur inspiré par le patient, depuis la chambre d'inhalation et à travers l'au moins une ouverture de sortie, jusque dans les narines du patient ; caractérisé en ce que l'au moins une ouverture de sortie présente un axe central incliné d'un angle compris entre 10° et 80° par rapport à l'axe longitudinal horizontal de la chambre d'inhalation.

Ainsi, l'invention permet de mieux cibler les zones inflammées en périphérie des cavités nasales, améliorant ainsi fortement l'efficacité du traitement de la rhinosinusite. L'invention utilise une interface nasale spécifique permettant, d'une part, la pénétration de l'embout nasal au-delà des poils du nez pour limiter sa filtration et, d'autre part, une étanchéité avec la narine pour assurer le fonctionnement correct de la chambre d'inhalation. L'utilisation d'une interface nasale en association avec la chambre d'inhalation permet d'assurer l'administration efficace d'aérosol produit par pMDI (« Pressurized Metered Dose Inhaler » en anglais) dans les cavités nasales du patient. La durée de génération d'aérosol dans la chambre d'inhalation est inférieure à 10 secondes, de préférence inférieure à 5 secondes.

En pratique, l'invention cumule les avantages de la nébulisation et de l'aérosol doseur. Les caractéristiques physiques de l'aérosol délivré sont équivalentes à celle produites par le nébuliseur. L'aérosol délivré au patient est constitué de fines particules ayant une vitesse réduite, compatible avec un dépôt dans les zones inflammatoires distales des cavités nasales. L'aérosol délivré est composé de particules sèches (évaporation des particules dans la chambre) limitant l'écoulement et la clairance du médicament depuis les cavités nasales vers la gorge puis l'estomac. L'invention est également remarquable en ce que la séance d'administration de l'aérosol ne durera que quelques secondes en comparaison d'une nébulisation de plus de 10 minutes, favorisant ainsi la compliance au traitement du patient.

Selon d'autres caractéristiques avantageuses de l'invention, prises isolément ou en combinaison :
- L'interface nasale comprend un embout simple, conçu pour être connecté de manière étanche à une seule narine du patient. L'embout simple comporte une unique ouverture de sortie.
- L'interface nasale comprend un embout double, conçu pour être connecté de manière étanche au deux narines du patient en même temps. L'embout double comporte deux ouvertures de sortie.
- L'ouverture de sortie de l'interface nasale présente un axe central incliné d'un angle égal à 30° par rapport à l'axe longitudinal horizontal de la chambre d'inhalation.
- Le corps comporte une paroi avant convergente s'étendant sur moins de 50% de la longueur de la chambre d'inhalation suivant l'axe longitudinal horizontal, et l'ouverture aval est située à l'extrémité du plus petit diamètre de la paroi avant convergente.
- L'ouverture aval est située sur l'axe longitudinal horizontal de la chambre d'inhalation.
- L'ouverture aval est centrée sur l'axe longitudinal horizontal de la chambre d'inhalation.
- L'interface nasale est constituée d'une seule pièce.
- L'interface nasale est constituée de plusieurs pièces.
- La valve inspiratoire est disposée au niveau de l'ouverture aval de la chambre d'inhalation.
- La valve inspiratoire est disposée au niveau de l'ouverture amont de la chambre d'inhalation.
- Le dispositif inhalateur comprend un générateur de son disposé entre la valve expiratoire et l'au moins une ouverture de sortie de l'interface nasale.
- Le générateur de son est configuré pour produire un son à une fréquence comprise entre 10 et 300 Hz, par exemple égale à 100 Hz.
- La chambre d'inhalation a une longueur comprise entre 50 mm et 400 mm, par exemple égale à 120 mm.
- La chambre d'inhalation a un volume compris entre 50 mL et 500 mL, par exemple égal à 250 mL.
- L'ouverture amont de la chambre d'inhalation est destinée à recevoir directement le générateur d'aérosol doseur.
- Au niveau de l'ouverture amont de la chambre d'inhalation, le dispositif inhalateur comporte un orifice longitudinal recevant la valve inspiratoire ; et un orifice latéral destiné à recevoir le générateur d'aérosol doseur.

L'invention a également pour objet un système de traitement, caractérisé en ce qu'il comprend :
- un dispositif inhalateur tel que mentionné ci-dessus ; et
- un générateur d'aérosol doseur, comprenant une cartouche contenant un médicament et connectée à la chambre d'inhalation, un actionnement manuel du générateur d'aérosol doseur provoquant la génération d'une dose d'aérosol dans la chambre d'inhalation pendant un temps inférieur à 10 secondes, de préférence inférieur à 5 secondes.

Selon un mode de réalisation préféré, la cartouche contient un médicament pour le traitement de la rhinosinusite, de préférence un corticoïde tel que du budesonide, de la beclométhasone ou de la fluticasone.

En alternative, la cartouche peut contenir un médicament pour le traitement d'autres pathologies, par exemple des antibiotiques, des anticorps, des bactériophages, des peptides, de l'insuline, des vaccins ou des antivirus.

Un procédé de mise en oeuvre d'un système de traitement tel que mentionné ci-dessus est ici décrit, le procédé comprenant les étapes suivantes :
a) une étape préparatoire consistant à connecter le dispositif inhalateur et le générateur d'aérosol doseur ;
b) une étape de positionnement consistant à positionner l'interface nasale sur le patient ;
c) une étape d'actionnement consistant à actionner le générateur d'aérosol doseur de sorte que l'aérosol est généré dans la chambre d'inhalation ; et
d) une étape d'administration consistant pour le patient au moins à inspirer à travers l'interface nasale, de sorte que l'aérosol est transporté depuis la chambre d'inhalation jusque dans les narines du patient.

L'étape d'administration comprend au moins une inspiration nasale. Avantageusement, l'inspiration nasale est précédée ou suivie par une expiration. L'inspiration nasale peut également être suivie de plusieurs cycles respiratoires (inspiration nasale + expiration) successifs.

L'étape d'actionnement de l'aérosol n'est pas nécessairement synchronisée à une phase respiratoire spécifique du patient.

De manière avantageuse, l'aérosol est délivré par le générateur d'aérosol doseur suivant l'axe longitudinal horizontal de la chambre d'inhalation.

L'invention sera mieux comprise à la lecture de la description qui va suivre, donnée uniquement à titre d'exemple non limitatif et faite en référence aux dessins annexés sur lesquels :
- la figure 1 est une coupe schématique d'un dispositif inhalateur non-conforme à l'invention, comprenant une chambre d'inhalation et une interface nasale ;
- la figure 2 est une vue de dessus de l'interface nasale de la figure 1, du type embout simple ;
- la figure 3 est une vue de dessus analogue à la figure 2, montrant une interface nasale du type embout double ;
- la figure 4 est une coupe analogue à la figure 1, montrant un système de traitement non-conforme à l'invention, comprenant un générateur d'aérosol doseur connecté au dispositif inhalateur ;
- la figure 5 est une coupe analogue à la figure 4, montrant un système de traitement conforme à un premier mode de réalisation de l'invention, dans lequel le dispositif inhalateur comporte une valve expiratoire ;
- la figure 6 est une coupe analogue à la figure 5, montrant un système de traitement conforme à un deuxième mode de réalisation de l'invention, dans lequel le dispositif inhalateur comporte un générateur de son ;
- les figures 7 et 8 sont des coupes analogues respectivement aux figures 1 et 4, montrant un système de traitement conforme à un troisième mode de réalisation de l'invention, dans lequel le dispositif inhalateur comporte une valve inspiratoire positionnée en amont du générateur d'aérosol doseur ;
- la figure 9 est un graphe montrant les résultats obtenus en mettant en oeuvre le système de traitement selon le premier mode de réalisation.

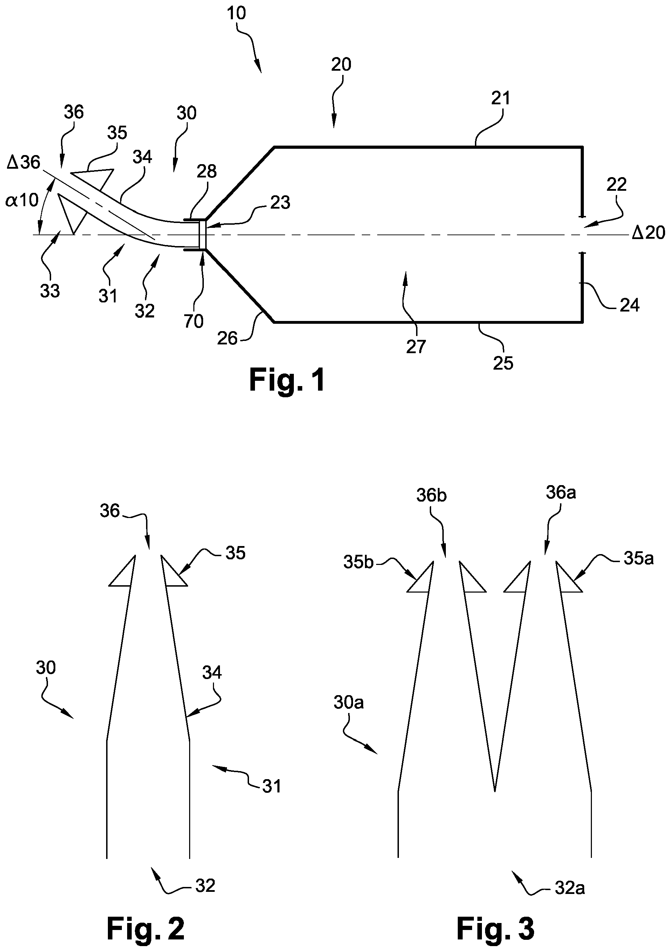

Sur la figure 1 est représenté un dispositif inhalateur 10 non-conforme à l'invention, conçue pour le traitement d'une rhinosinusite chez un patient.

Le dispositif 10 est configuré pour être utilisé horizontalement. Le dispositif 10 comprend une chambre d'inhalation 20, une interface nasale 30 et une valve inspiratoire unidirectionnelle 70. Le dispositif 10 est conçu pour l'évaporation et le freinage de l'aérosol dans la chambre 20, puis l'inspiration de l'aérosol par le patient à travers l'interface nasale 30.

La chambre d'inhalation 20 comprend un corps creux 21 pourvu de deux ouvertures 22 et 23. Le corps 21 comprend une paroi arrière plane 24, une paroi intermédiaire cylindrique 25 et une paroi avant tronconique convergente 26. La chambre 20 présente un axe longitudinal horizontal Δ20.

L'ouverture 22 est formée au travers de la paroi arrière 24 et peut donc être qualifiée d'ouverture arrière ou amont. L'ouverture 23 est située à l'extrémité du plus petit diamètre de la paroi tronconique 26 et peut donc être qualifiée d'ouverture avant ou aval.

L'ouverture amont 22 est prévue pour recevoir un générateur d'aérosol doseur pressurisé 40, comme montré à la figure 4. L'ouverture aval 23 est connectée à l'interface nasale 30. La valve 70 est positionnée entre la chambre 20 et l'interface 30, au niveau de l'ouverture 23.

Le corps 21 a une longueur de 120 mm entre les ouvertures 22 et 23. La paroi 25 présente un diamètre de 60 mm et une longueur de 100 mm. La paroi 26 présente un diamètre de 22 mm dans sa plus petite section, au niveau de l'ouverture 23, et une longueur de 20 mm. La paroi 26 s'étend sur moins de 50% de la longueur de la chambre 20, plus précisément sur 17 % de la longueur de la chambre 20 sur l'exemple de la figure 1. Le corps 21 délimite un volume interne 27 de 250 mL environ, avec une forme convergente depuis la paroi 24 jusqu'à l'ouverture 23. Du fait de la forme du corps 21, l'évaporation de l'aérosol est facilitée sans perte importante de particules par impaction dans la chambre 20. La vitesse d'injection des particules étant forte, elle nécessite une distance suffisante et une forme adaptée pour assurer leur freinage sans impaction sur les parois internes de la chambre 20.

Avantageusement, le corps 21 peut comporter un tube avant 28 de forme cylindrique, connecté à la paroi 26 au niveau de l'ouverture 23. Le tube 28 facilite le raccordement de l'interface nasale 30 à l'ouverture 23.

L'interface nasale 30 montrée sur les figures 1 et 2 est du type embout nasal simple. L'interface 30 est configurée pour rendre étanche l'embout nasal en regard de la narine afin de guider l'aérosol inspiré par le patient, depuis la chambre 20 jusque dans la narine du patient. L'interface 30 est réalisée dans un matériau souple, ce qui est plus confortable pour le patient, et permet de plus de s'affranchir des mouvements du patient.

L'interface 30 comprend un conduit 31 qui s'étend entre une extrémité amont 32 et une extrémité aval 33. Le conduit 31 comporte une paroi tubulaire souple 34. A l'extrémité 32, le conduit 31 est introduit dans le tube 28. Le conduit 31 est ainsi raccordé à l'ouverture 23 en formant une liaison étanche entre le tube 28 et la paroi 34 de l'interface 30. A l'extrémité 33, l'interface 30 est pourvue d'un embout 35 prévu pour être introduit de manière étanche dans la narine d'un patient. Une ouverture de sortie 36 est ménagée à l'embouchure du conduit 31 et de l'embout 35.

L'ouverture 36 présente un axe central Δ36, incliné d'un angle α10 égal à 30° par rapport à l'axe longitudinal Δ20 de la chambre 20. En alternative, l'angle α10 peut être compris entre 10° et 80°.

La mise en oeuvre d'un angle α10 spécifique d'administration de l'aérosol dans les cavités nasales permet de cibler les zones anatomiques d'intérêt. Plus précisément, cet angle α10 permet la pénétration d'aérosol dans les zones supérieures des cavités nasales.

Pour contre-exemple, l'utilisation d'un angle α10 de 0° aurait pour effet de faire pénétrer l'aérosol le long du plancher des cavités nasales. L'aérosol ne pénétrerait pas dans les zones supérieures des cavités nasales, zone d'intérêt pour le traitement des rhinosinutes.

Sur la figure 3 est représentée une interface nasale 30a, du type embout double. Cette interface nasale 30a comporte deux embouts 35a et 35b, chacun muni d'une ouverture de sortie 36a et 36b. Les deux embouts 35a et 35b sont reliés entre eux de manière symétrique à l'extrémité 32a.

Sur la figure 4 est représenté un système de traitement 1 non-conforme à l'invention.
Le système 1 comprend le dispositif 10 de la figure 1 et le générateur d'aérosol doseur pressurisé 40.

Le générateur 40 comprenant une cartouche 50 contenant un médicament et un boitier 60 recevant la cartouche 50. Pour soigner une rhinosinusite, le médicament inclut de préférence du budesonide, par exemple une dose de 200 µg de budesonide pour un adulte et 100 µg pour un enfant.

La cartouche 50 comprend un corps 51 et une tête 52 munie d'un orifice de pulvérisation 53. Une pression sur la tête 52 pompe le médicament contenu dans le corps 51 et le projette hors de la tête 52 par l'orifice de pulvérisation 53.

Le boitier 60 comprend un compartiment 61 délimité par une paroi cylindrique 64 et une paroi inférieure plane 65. Le compartiment 61 est prévu pour recevoir la cartouche 50. Le boitier comporte deux ouvertures 62 et 63. Le boitier 60 comporte également un embout 66 délimité par une paroi cylindrique d'axe horizontal. L'embout 66 est placé en saillie de la paroi 64. Le compartiment 61 et l'embout 66 communiquent l'un avec l'autre.

L'ouverture 62 est ménagée à l'extrémité supérieure du compartiment 61 et peut donc être qualifiée d'ouverture supérieure ou amont. L'ouverture 63 est ménagée à l'extrémité avant de l'embout 66 et peut donc être qualifiée d'ouverture avant ou aval.

La cartouche 50 est introduite dans le boitier 60 à travers l'ouverture 62, avec la tête 52 en bas. La cartouche 50 est introduite dans le compartiment 61, avec la tête 52 venant reposer contre la paroi 65. L'orifice 53 est orienté vers l'ouverture 63 de l'embout 66. Le corps 51 dépasse du compartiment 61 à travers l'ouverture 62. La cartouche 50 est ainsi accessible hors du boitier 60. L'ouverture 62 reste libre, ouverte sur l'air ambiant.

Le générateur 40 est un aérosol doseur pressurisé (pMDI) pour une administration du médicament par voie orale, connu en soit et déjà disponible dans le commerce. L'embout 66 est dimensionné pour être introduit dans la bouche du patient, et peut donc être qualifié d'embout buccal. Le générateur 40 est configuré pour être utilisé verticalement (la cartouche 50 devant être positionnée de manière verticale) et générer un aérosol A1 selon une direction horizontale à travers l'embout 66.

Dans cet exemple, l'embout 66 est reçu à travers l'ouverture 22 de la chambre d'inhalation 20. Connectés l'un à l'autre, le dispositif 10 et le générateur 40 constituent ainsi le système de traitement 1.

En pratique, une pression manuelle exercée sur la cartouche 50 comprime sa tête 52 contre la paroi 65 du boitier 60. Une dose d"aérosol A1 est alors projetée en quantité déterminée par l'orifice 53, dans l'embout 66, jusqu'à l'ouverture 63.

L'aérosol A1 quitte alors le dispositif 40 et pénètre dans la chambre 20. Compte tenu des formes et dimensions de la chambre 20, l'aérosol A1 produit par le générateur 40 est freiné et s'évapore dans le volume 27 suivant l'axe longitudinal horizontal Δ20. Ainsi, lorsque le générateur 40 est actionné manuellement, une dose d'aérosol A1 est produite dans la chambre 20.

A ce stade, le patient peut inspirer à travers l'interface 30, avec l'embout 35 positionné dans une de ses narines. Le patient doit boucher manuellement son autre narine à l'air libre, avec une pression de son doigt sur cette même narine. L'air A2 inspiré par le patient traverse successivement les ouvertures 62, 63 et 22, puis transporte l'aérosol A1 de la chambre d'inhalation 20 jusque dans la narine du patient via la valve inspiratoire 70 et l'interface nasale 30, en traversant successivement les ouvertures 23 et 36.

Ainsi, l'aérosol A1 administré au patient grâce au dispositif 10 est constitué de fines particules ayant une faible vitesse. Le dispositif 10 permet de réaliser un dépôt distal de l'aérosol A1 dans les cavités nasales, ce qui améliore fortement l'efficacité du traitement.

Lors de la phase expiratoire du patient, le patient ne peut qu'expirer par la bouche. L'aérosol A1 résiduel restant dans la chambre 20 est stocké pour l'inspiration suivante par le patient. Par exemple, dans le cas d'un enfant inspirant un volume de 100 mL, plusieurs cycles respiratoires sont nécessaires pour vider totalement la chambre 20 d'un volume 27 de 250 mL.

Il est à noter que la pression manuelle exercée sur le corps 51 de la cartouche 50 pour projeter l'aérosol A1 dans la chambre 20 peut être réalisée non limitativement durant la phase inspiratoire ou expiratoire du patient compte tenu de la capacité de stockage du dispositif 10. Un meilleur effet est néanmoins obtenu lorsque la pression manuelle sur le corps 51 de la cartouche 50 est exercée durant la phase inspiratoire.

Il est à noter également que dans cet exemple, le générateur 40 permet le libre passage de l'air. En conséquence, le positionnement de la valve inspiratoire unidirectionnelle 70 sur le trajet de l'aérosol A1 est nécessaire afin d'assurer la circulation de l'air A2 depuis l'extérieur du système de traitement 1 vers le patient et empêcher l'expiration nasale du patient par l'interface nasale 30, ce qui aurait pour conséquence l'échappement de l'aérosol A1 hors du système 1.

A ce stade, on note que les directions « amont » et « aval » sont définies au sein du système 1 par rapport aux flux A1 et A2.

Des modes de réalisation d'un système de traitement 1 et d'un dispositif inhalateur 10 conformes à l'invention sont montrés aux figures 5 à 8. Certains éléments constitutifs du système 1 et du dispositif 10 sont comparables à ceux de l'exemple décrit ci-dessus et, dans un but de simplification, portent les mêmes références numériques. Seules les différences avec cet exemple sont décrites ci-après.

Sur la figure 5, le dispositif 10 comporte une interface 30a du type double embout, une valve inspiratoire 70 et une valve expiratoire 80. Chacune des ouvertures 36a et 36b présente un axe central, incliné d'un angle α10 égal à 30° par rapport à l'axe longitudinal Δ20 de la chambre 20.

La valve 70 est positionnée entre la chambre 20 et l'interface 30a, au niveau de l'ouverture 23. La valve 80 est positionnée sur le tube 28, en aval de la valve 70. Plus précisément, la valve 80 est positionnée entre la valve 70 et les ouvertures 36a et 36b, en étant plus rapprochée de la valve 70 que des ouvertures 36a et 36b.

Dans un premier temps, le patient expire de l'air A0 à travers l'interface 30a. La valve inspiratoire 70 est fermée et la valve expiratoire 80 est ouverte. L'air A0 expiré par le patient traverse l'interface 30a puis s'échappe de la valve expiratoire 80. L'air A0 ne traverse pas la chambre 20. Ainsi, l'aérosol A1 reste stocké dans la chambre 20.

Dans un second temps, le patient inspire à travers l'interface 30a. La valve inspiratoire 70 est ouverte et la valve expiratoire 80 est fermée. L'air A2 inspiré par le patient traverse successivement les ouvertures 62, 63 et 22, puis transporte l'aérosol A1 de la chambre d'inhalation 20 jusque dans les narines du patient via l'interface nasale 30a, en traversant successivement la valve 70, l'ouverture 23, puis les ouvertures 36a et 36b simultanément.

Ainsi, l'aérosol A1 administré au patient grâce au dispositif 10 est constitué de fines particules ayant une faible vitesse. Le dispositif 10 permet de réaliser un dépôt distal de l'aérosol A1 dans les cavités nasales.

Dans cette configuration, l'aérosol A1 peut être projeté dans la chambre 20 durant la phase inspiratoire ou expiratoire du patient compte tenu de l'effet stockage du dispositif 10. Le patient peut également inspirer par le nez et expirer par le nez. Il peut également expirer par la bouche sans fermer manuellement une de ses deux narines.

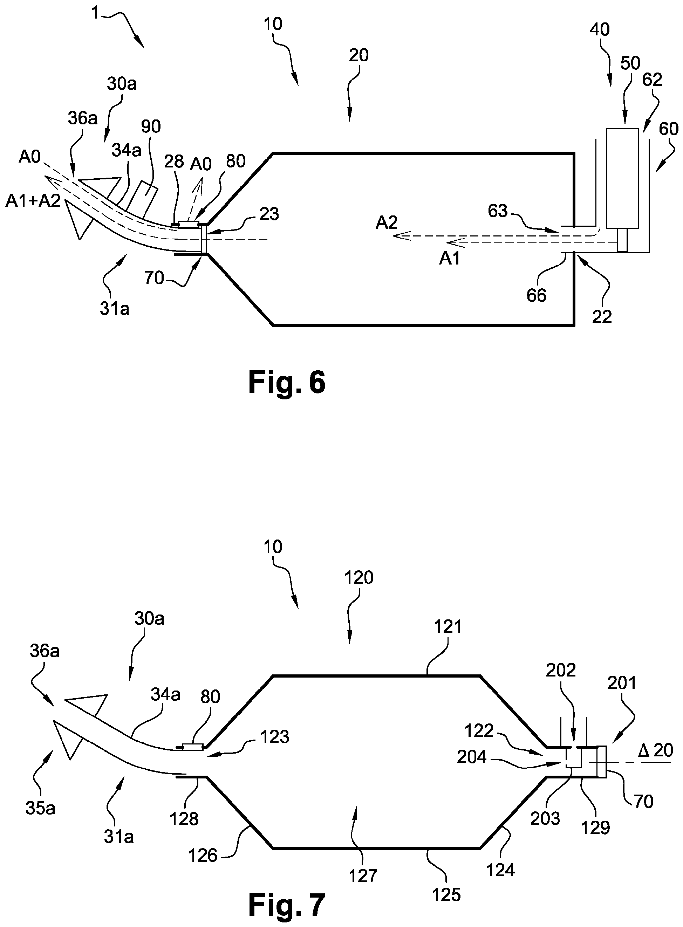

Sur la figure 6, le dispositif 10 comprend un générateur de son 90 équipant l'interface 30a en aval de la valve 80. Plus précisément, le générateur de son 90 est positionné contre la paroi de l'interface nasale 30a, entre la valve expiratoire 80 et les ouvertures de sortie 36a et 36b. Le générateur de son 90 est configuré pour produire un son à une fréquence comprise entre 10 et 300 Hz. De préférence, cette fréquence est égale à 100 Hz.

Le générateur de son 90 est mis en fonctionnement lors du traitement. Ainsi, l'aérosol A1 administré au patient grâce au dispositif 10 est constitué de fines particules ayant une faible vitesse, en association avec un son d'une fréquence de 100 Hz. L'aérosol A1 appelé « aérosol sonique » qui pénètre dans les cavités nasales avec le flux d'air A2 est soumis à une pression acoustique au niveau de l'ostium des sinus maxillaires. Le dispositif 10 permet de réaliser un dépôt distal de l'aérosol A1 dans les cavités nasales, et plus particulièrement au niveau des sinus maxillaires, ce qui améliore encore l'efficacité du traitement.

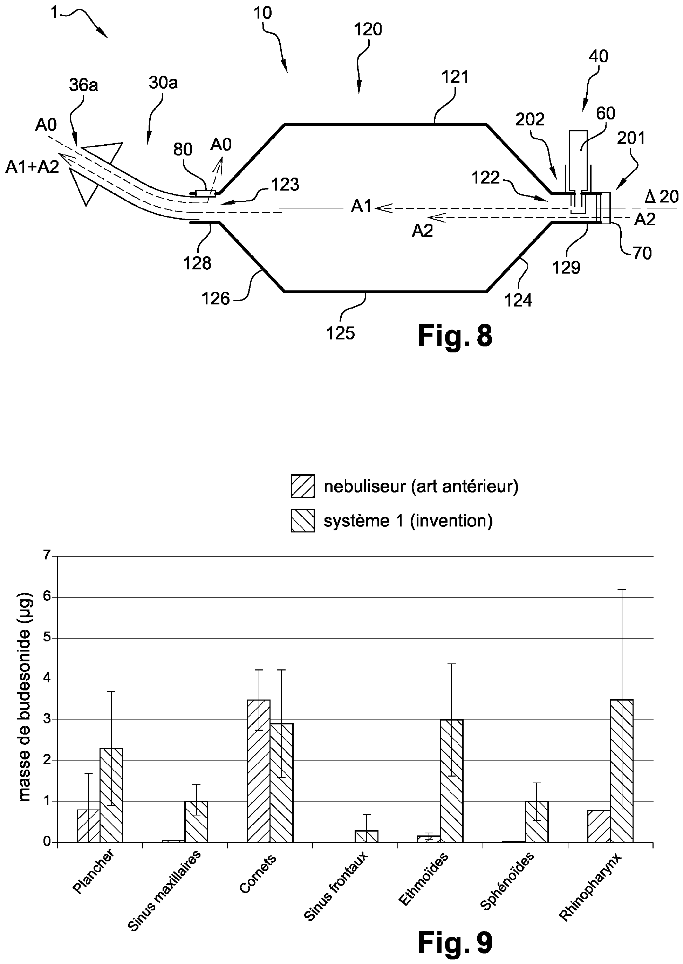

Sur les figures 7 et 8 est représenté un autre mode de réalisation de l'invention.

Le dispositif 10 comprend une chambre d'inhalation 120, une interface nasale 30a du type embout double, une valve inspiratoire 70 et une valve expiratoire 80. La chambre d'inhalation 120 a un corps 121 de forme différente du corps 21 décrit plus haut. Le générateur 40 comporte une cartouche 50 devant être utilisée de manière verticale, mais pas de boitier 60.

Le corps 121 est pourvu d'une ouverture amont 122 et d'une ouverture aval 123. Le corps 121 comprend une paroi arrière tronconique divergente 124, une paroi intermédiaire cylindrique 125 et une paroi avant tronconique convergente 126. Le corps 121 délimite un volume interne 27 de 250 mL environ.

L'ouverture 122 est formée à l'extrémité de plus petit diamètre de la paroi tronconique 124 et peut donc être qualifiée d'ouverture arrière ou amont. L'ouverture 123 est formée à l'extrémité de plus petit diamètre de la paroi tronconique 26 et peut donc être qualifiée d'ouverture avant ou aval.

Le corps 121 comporte un tube avant 128 de forme cylindrique, connecté à la paroi 126 au niveau de l'ouverture 123. Le tube 128 comprend la valve expiratoire 80 sur sa partie latérale et facilite le raccordement de l'interface nasale 30 à l'ouverture 123.

Le corps 121 comporte également un tube arrière 129 de forme cylindrique, connecté à la paroi 124 au niveau de l'ouverture 122. Le tube 129 est pourvu de deux orifices 201 et 202. L'orifice 201 est formé suivant l'axe longitudinal Δ20 de la chambre 120 et peut donc être qualifiée d'orifice longitudinal. L'orifice 202 est formé sur un côté du tube 129 et peut donc être qualifiée d'orifice latéral. Dans le tube 129 est formé un élément support 203 recevant la tête 52 de la cartouche 50. L'élément support 203 comporte un orifice 204 dirigé suivant l'axe longitudinal Δ20 de la chambre 120. Les orifices 202 et 204 communiquent entre eux.

Dans cette configuration, l'aérosol A1 peut être délivré dans la chambre 120 durant la phase inspiratoire ou expiratoire du patient, compte tenu de l'effet stockage de cette chambre 120.

Dans un premier temps, le patient expire de l'air A0 à travers l'interface nasale 30a. La valve inspiratoire 70 est fermée et la valve expiratoire 80 est ouverte. L'air A0 expiré par le patient traverse l'interface 30a puis s'échappe par la valve expiratoire 80. L'air A0 ne traverse pas la chambre 120. Ainsi, l'aérosol A1 reste stocké dans le volume 127 de la chambre 120.

Dans un deuxième temps, le patient inspire à travers l'interface 30a. La valve inspiratoire 70 est ouverte et la valve expiratoire 80 est fermée. L'air A2 inspiré par le patient traverse successivement les ouvertures 201, 122, 123, puis transporte l'aérosol A1 préalablement délivré dans le volume 127, depuis la chambre d'inhalation 120 jusque dans les narines du patient via l'interface nasale 30a, puis simultanément via les ouvertures 36a et 36b.

Ainsi, l'aérosol A1 administré au patient grâce au dispositif 10 est constitué de fines particules ayant une faible vitesse. Le dispositif 10 permet de réaliser un dépôt distal de l'aérosol A1 dans les cavités nasales. Le patient peut également inspirer par le nez et expirer par le nez ou par la bouche. L'aérosol A1 ne traverse aucune valve (en particulier la valve inspiratoire 70, limitant ainsi le piégeage des particules dans la valve 70).

Il est à noter également que dans ce mode de réalisation, le générateur 40 est étanche et ne permet pas le passage de l'air. En conséquence, la valve inspiratoire unidirectionnelle 70 peut être placée sur l'ouverture amont 201 afin d'assurer la circulation de l'air A2 depuis l'extérieur du système de traitement 1 vers le patient et empêcher l'expiration nasale du patient dans le système 1, ce qui aurait pour conséquence l'échappement de l'aérosol A1 hors du système 1 (avant son inspiration par l'interface nasale 30).

Sur la figure 9 est représenté un graphe montrant les résultats obtenus en mettant en oeuvre le système de traitement 1 selon le premier mode de réalisation, correspondant à la figure 5.

En abscisses sont représentées différentes zones des voies respiratoires supérieures du patient, à savoir le plancher, les sinus maxillaires, les cornets, les sinus frontaux, les ethmoïdes, les sphénoïdes et le rhinopharynx. En ordonnée est représentée la masse de budesonide (µg) déposée dans ladite zone.

Pour chaque zone, le bâton de gauche correspond aux résultats obtenus avec un nébuliseur classique à visée nasale et chargé avec 1mg/4mL de budésonide, tandis que le bâton de droite correspond aux résultats obtenus avec le système 1 selon l'invention et utilisé avec deux doses de 100 µg administrés avec un pMDI de budésonide.

On constate que le dépôt de budesonide est nettement plus efficace avec le système 1 en comparaison avec le nébuliseur, et ceci pour une dose 5 fois moins importante avec le système 1 que le nébuliseur. La durée de l'inhalation avec le nébuliseur était de 10 minutes, alors qu'elle n'était que de 30 secondes avec l'invention.

Quel que soit le mode de réalisation, la présente invention cumule les avantages des sprays et des nébuliseurs. Le système 1 permet l'administration de particules équivalentes aux particules produites par les systèmes de nébulisation, en quelques secondes d'inhalation. L'administration du traitement est donc rapide et efficace pour cibler les zones distales des cavités nasales. L'invention est particulièrement avantageuse pour le traitement des petits enfants, pour lesquels la compliance au traitement et l'accès aux petites zones des cavités nasales avec les sprays sont difficiles. Egalement, l'invention a pour avantage la compatibilité du dispositif 10 avec les générateurs doseurs 40 pulmonaires pour le traitement nasal.

En pratique, la comparaison entre les quantités délivrées de budesonide par aérosol doseur et par nébulisation démontre que l'administration d'une dose de 200 µg de budesonide par aérosol doseur en quelques secondes d'inhalation est équivalente à 1 mg de budesonide administré par nébulisation en dix minutes.

Le système de traitement 1, le dispositif inhalateur 10 et le générateur 40 peuvent être conformés différemment des figures 1 à 8 sans sortir du cadre de l'invention tel que défini par les revendications annexées.

En variante non représentée, la chambre d'inhalation 20 / 120 peut être plus ou moins allongée et/ou présenter un diamètre plus ou moins large.

Selon une autre variante non représentée, l'ouverture amont 22 / 122 peut être formée au travers du corps 21 / 121 ailleurs que suivant l'axe longitudinal Δ20 de la chambre d'inhalation 20 / 120.

Selon une autre variante non représentée, l'interface nasale 30 / 30a peut être constituée de plusieurs pièces, notamment constituées de matériaux différents.

Selon une autre variante non représentée, le générateur 40 peut être du type aérosol doseur de liquide pressurisé sans gaz propulseur (« soft mist inhaler » en anglais, par exemple dispositif commercialisé sous la marque « Respimat », pompe nasale produisant de fines particules).

Selon une autre variante, le générateur 40 (et en particulier la cartouche 50) peut être placé en aval de la chambre d'inhalation 120, et plus précisément entre la valve expiratoire 80 et la chambre d'inhalation 120, et plus précisément encore entre la valve expiratoire 80 et l'extrémité de la plus petite section de la partie tronconique 126, avec une orientation du spray de l'aérosol A1 produit vers la partie amont de la chambre 120.

En outre, les caractéristiques techniques des différents modes de réalisation et variantes mentionnés ci-dessus peuvent être, en totalité ou pour certaines d'entre elles, combinées entre elles. Ainsi, le système 1, le dispositif 10 et le générateur 40 peuvent être adaptés en termes de coût, d'ergonomie, de fonctionnalités et de performance.

## Revendications

1. Dispositif inhalateur (10), destiné à recevoir un générateur d'aérosol doseur (40), pour le traitement des pathologies nasales chez un patient,
le dispositif inhalateur (10) étant configuré pour être utilisé horizontalement et comprenant :
- une chambre d'inhalation (20 ; 120) présentant un axe longitudinal horizontal (Δ20),
- une interface nasale (30 ; 30a) du type embout nasal comportant au moins une ouverture de sortie (36 ; 36a, 36b),
- une valve inspiratoire (70) unidirectionnelle, et
- une valve expiratoire (80) disposée entre la valve inspiratoire (70) et l'au moins une ouverture de sortie (36 ; 36a, 36b) de l'interface nasale (30 ; 30a) ;
dans lequel la chambre d'inhalation (20 ; 120) comporte :
- un corps (21 ; 121) allongé suivant l'axe longitudinal horizontal (Δ20) et délimitant un volume intérieur (27 ; 127) pour l'évaporation et le freinage d'un aérosol (A1) délivré par le générateur d'aérosol doseur (40),
- une ouverture aval (23 ; 123) connectée à l'interface nasale (30 ; 30a), et
- une ouverture amont (22 ; 122) destinée à faire pénétrer de l'air extérieur (A2) dans le volume intérieur (27 ; 127) de la chambre d'inhalation (20 ; 120) pour assurer le transport efficace de l'aérosol (A1) vers l'ouverture aval (23 ; 123) ;
dans lequel la valve inspiratoire (70) est disposée au niveau de l'ouverture aval (23) ou de l'ouverture amont (122) de la chambre d'inhalation (20 ; 120) ;
et dans lequel l'interface nasale (30 ; 30a) est configurée pour guider l'aérosol (A1) et l'air extérieur (A2) inspiré par le patient, depuis la chambre d'inhalation (20 ; 120) et à travers l'au moins une ouverture de sortie (36 ; 36a, 36b), jusque dans les narines du patient ;
**caractérisé en ce que** l'au moins une ouverture de sortie (36 ; 36a, 36b) présente un axe central (Δ36) incliné d'un angle (α10) compris entre 10° et 80° par rapport à l'axe longitudinal horizontal (Δ20) de la chambre d'inhalation (20 ; 120).

2. Dispositif inhalateur (10) selon la revendication précédente, **caractérisé en ce que** l'interface nasale (30a) comprend un embout double, conçu pour être connecté de manière étanche au deux narines du patient en même temps.

3. Dispositif inhalateur (10) selon l'une des revendications précédentes, **caractérisé en ce que** le corps (21 ; 121) comporte une paroi avant convergente (26 ; 126) s'étendant sur moins de 50% de la longueur de la chambre d'inhalation (20 ; 120) suivant l'axe longitudinal horizontal (Δ20), et **en ce que** l'ouverture aval (23 ; 123) est située à l'extrémité du plus petit diamètre de la paroi avant convergente (26 ; 126).

4. Dispositif inhalateur (10) selon l'une des revendications précédentes, **caractérisé en ce qu'**il comprend un générateur de son (90) disposé entre la valve expiratoire (80) et l'au moins une ouverture de sortie (36 ; 36a, 36b) de l'interface nasale (30 ; 30a).

5. Dispositif inhalateur (10) selon la revendication 4, **caractérisé en ce que** le générateur de son (90) est configuré pour produire un son à une fréquence comprise entre 10 et 300 Hz, par exemple égale à 100 Hz.

6. Dispositif inhalateur (10) selon l'une des revendications précédentes, **caractérisé en ce que** l'interface nasale (30 ; 30a) est réalisée dans un matériau souple.

7. Dispositif inhalateur (10) selon l'une des revendications précédentes, **caractérisé en ce que** la chambre d'inhalation (20 ; 120) a une longueur comprise entre 50 mm et 400 mm.

8. Dispositif inhalateur (10) selon l'une des revendications précédentes, **caractérisé en ce que** la chambre d'inhalation (20 ; 120) a un volume (27 ; 127) compris entre 50 mL et 500 mL.

9. Système de traitement (1), **caractérisé en ce qu'**il comprend :
- un dispositif inhalateur (10) selon l'une des revendications 1 à 8 ; et
- un générateur d'aérosol doseur (40), comprenant une cartouche (50) contenant un médicament et connectée à la chambre d'inhalation (20 ; 120), un actionnement manuel du générateur d'aérosol doseur (40) provoquant la génération d'une dose d'aérosol (A1) dans la chambre d'inhalation (20 ; 120) pendant un temps inférieur à 10 secondes, de préférence inférieur à 5 secondes.

10. Système de traitement (1) selon la revendication 9, **caractérisé en ce que** la cartouche (50) contient un médicament pour le traitement de la rhinosinusite, de préférence un corticoïde.

11. Système de traitement (1) selon l'une des revendications 9 ou 10, **caractérisé en ce que** l'ouverture amont (22) de la chambre d'inhalation (20) reçoit directement le générateur d'aérosol doseur (40).

12. Système de traitement (1) selon l'une des revendications 9 ou 10, **caractérisé en ce qu'**au niveau de l'ouverture amont (122) de la chambre d'inhalation (120), le dispositif inhalateur (10) comporte :
- un orifice longitudinal (201) recevant la valve inspiratoire (70) ; et
- un orifice latéral (202) destiné à recevoir le générateur d'aérosol doseur (40).

## Patentansprüche

1. Inhalator (10), der zur Aufnahme einer Dosier-Aerosolpackung (40) zur Behandlung nasaler Pathologien bei einem Patienten bestimmt ist,
wobei der Inhalator (10) konfiguriert ist, um horizontal eingesetzt zu werden und umfasst:
- eine Inhalationskammer (20, 120) mit einer horizontalen Längsachse (Δ20),
- ein Nasenzwischenstück (30; 30a) vom Typ Nasensonde mit mindestens einer Ausgangsöffnung (36; 36a, 36b),
- ein Einatemventil (70) in einer Richtung, und
- ein Ausatemventil (80), das zwischen dem Einatemventil (70) und der mindestens einen Ausgangsöffnung (36; 36a, 36b) des Nasenzwischenstücks (30; 30a) angeordnet ist,
bei dem die Inhalationskammer (20, 120) umfasst:
- ein Gehäuse (21, 121), längsverlaufend entlang der horizontalen Längsachse (Δ20), das einen Innenraum (27, 127) umschließt, zur Verdampfung und Abbremsung eines Aerosols (A1), das von der Dosier-Aerosolpackung (40) abgegeben wird,
- eine dahinter liegende Öffnung (23, 123), die mit dem Nasenzwischenstück (30; 30a) verbunden ist, und
- eine davor liegende Öffnung (22, 122), durch die die Außenluft (A2) in den Innenraum (27, 127) der Inhalationskammer (20, 120) gelangen soll, um den wirksamen Transport des Aerosols (A1) zur dahinter liegenden Öffnung (23, 123) sicherzustellen;
in dem das Einatemventil (70) in Höhe der dahinter liegenden Öffnung (23) oder der davor liegenden Öffnung (122) der Inhalationskammer (20, 120) angeordnet ist,
und bei dem das Nasenzwischenstück (30; 30a) konfiguriert ist, um das Aerosol (AI) und die Außenluft (A2), die vom Patienten eingeatmet werden, von der Inhalationskammer (20, 120) und durch mindestens eine Ausgangsöffnung (36; 36a, 36b) bis in die Nasenlöcher des Patienten zu leiten;
**dadurch gekennzeichnet, dass** mindestens eine Ausgangsöffnung (36; 36a, 36b) eine zentrale Achse (Δ36) aufweist, die um einen Winkel (α10) zwischen 10° und 80° gegenüber der horizontalen Längsachse (Δ20) der Inhalationskammer (20, 120) geneigt ist.

2. Inhalator (10) gemäß dem vorhergehenden Anspruch, **dadurch gekennzeichnet, dass** das Nasenzwischenstück (30a) eine doppelte Sonde umfasst, die entworfen ist, um dicht mit den beiden Nasenlöchern des Patienten gleichzeitig verbunden zu werden

3. Inhalator (10) nach einem der vorhergehenden Ansprüche, **dadurch gekennzeichnet, dass** das Gehäuse (21, 121) eine konvergierende Vorderwand (26, 126) enthält, die über mindestens 50% der Länge der Inhalationskammer (20 , 120) entlang der horizontalen Längsachse (Δ20) verläuft und dass sich die dahinter liegende Öffnung (23, 123) am Ende des kleinsten Durchmessers der konvergierenden Vorderwand (26, 126) befindet.

4. Inhalator (10) nach einem der vorhergehenden Ansprüche, **dadurch gekennzeichnet, dass** er einen Tongenerator (90) enthält, der zwischen dem Ausatemventil (80) und der mindestens einen Ausgangsöffnung (36 ; 36a, 36b) des Nasenzwischenstücks (30 ; 30a) angeordnet ist.

5. Inhalator (10) gemäß Anspruch 4, **dadurch gekennzeichnet, dass** der Tongenerator (90) konfiguriert ist, um einen Ton mit einer Frequenz zwischen 10 und 300 Hz, zum Beispiel gleich 100 Hz zu erzeugen.

6. Inhalator (10) nach einem der vorhergehenden Ansprüche, **dadurch gekennzeichnet, dass** das Nasenzwischenstück (30; 30a) aus einem flexiblen Material ausgeführt ist.

7. Inhalator (10) nach einem der vorhergehenden Ansprüche, **dadurch gekennzeichnet, dass** die Inhalationskammer (20, 120) eine Länge zwischen 50 mm und 400 mm hat.

8. Inhalator (10) nach einem der vorhergehenden Ansprüche, **dadurch gekennzeichnet, dass** die Inhalationskammer (20, 120) ein Volumen (27, 127) zwischen 50 mL und 500 mL hat

9. Behandlungssystem (1), **dadurch gekennzeichnet, dass** es umfasst:
- einen Inhalator (10), nach einem der Ansprüche 1 bis 8; und
- eine Dosier-Aerosolpackung (40) mit einer Kartusche (50), die ein Medikament enthält und mit der Inhalationskammer (20, 120) verbunden ist, eine manuelle Betätigung der Dosier-Aerosolpackung (40), die die Erzeugung einer Aerosol-Dosis (A1) in der Inhalationskammer (20, 120) während einer Zeit von unter 10 Sekunden, noch besser unter 5 Sekunden verursacht.

10. Behandlungssystem (1) gemäß Anspruch 9, **dadurch gekennzeichnet, dass** die Kartusche (50) ein Medikament zur Behandlung der Rhinosinusitis enthält, am besten ein Kortikoid.

11. Behandlungssystem (1) nach einem der Ansprüche 9 oder 10, **dadurch gekennzeichnet, dass** die Dosier-Aerosolpackung (40) direkt in die davor liegende Öffnung (22) der Inhalationskammer (20) eingesetzt wird.

12. Behandlungssystem (1) nach einem der Ansprüche 9 oder 10, **dadurch gekennzeichnet, dass** in Höhe der davor liegenden Öffnung (122) der Inhalationskammer (120) der Inhalator (10) umfasst:
- eine Längsöffnung (201) zur Aufnahme des Einatemventils (70); und
- eine seitliche Öffnung (202) zur Aufnahme der Dosier-Aerosolpackung (40).

## Claims

1. Inhaler device (10) designed to accommodate a dosing aerosol generator (40) for the treatment of nasal pathologies in a patient,
with the inhaler device (10) being configured to be used horizontally and incorporating:
• one inhalation chamber (20; 120) having one horizontal longitudinal axis (Δ20);
• one nasal interface (30; 30a) of the nasal end piece type, incorporating at least one outlet opening (36; 36a; 36b),
• one one-way inspiration valve (70), and
• one expiration valve (80) located between the inspiration valve (70) and the outlet opening (36; 36a; 36b) - of which there is at least one - of the nasal interface (30; 30a);
in which the inhalation chamber (20; 120) incorporates:
• an extended body (21; 121) in the horizontal longitudinal axis (Δ20), bounding an interior volume (27; 127) for the evaporation and the braking of an aerosol (A1) delivered by the dosing aerosol generator (40),
• one downstream opening (23; 123) connected to the nasal interface (30; 30a), and
• one upstream opening (22; 122) designed to cause outside air (A2) to enter the interior volume (27; 127) of the inhalation chamber (20; 120) to ensure the efficient transfer of the aerosol (A1) to the downstream opening (23; 123);
in which the inspiration valve (70) is positioned at the downstream opening (23) or the upstream opening (122) of the inhalation chamber (20; 120);
and in which the nasal interface (30; 30a) is configured to guide the aerosol (A1) and the outside air (A2) inhaled by the patient from the inhalation chamber (20; 120) and through the outlet opening (36; 36a; 36b) - of which there is at least one - as far as the patient's nostrils;
**characterized by** the fact that the outlet opening (36; 36a; 36b) - of which there is at least one - has a central axis (Δ36) inclined at an angle (α10) of between 10° and 80° in relation to the horizontal longitudinal axis (Δ20) of the inhalation chamber (20; 120).

2. Inhaler device (10) in accordance with the preceding claim, **characterized by** the fact that the nasal interface (30a) incorporates a double end piece designed to be connected impermeably to both the patient's nostrils at the same time.

3. Inhaler device (10) in accordance with one of the preceding claims, **characterized by** the fact that the body (21; 121) incorporates a convergent front wall (26; 126) extending over at least 50% of the length of the inhalation chamber (20; 120) in the horizontal longitudinal axis (Δ20), and by the fact that the downstream opening (23; 123) is located at the end of the smallest diameter of the convergent front wall (26; 126).

4. Inhaler device (10) in accordance with one of the preceding claims, **characterized by** the fact that it incorporates a sound generator (90) positioned between the expiration valve (80) and the outlet opening (36; 36a; 36b) - of which there is at least one - of the nasal interface (30; 30a).

5. Inhaler device (10) in accordance with claim 4, **characterized by** the fact that the sound generator (90) is configured to produce a sound at a frequency of between 10 and 300 Hz - 100 Hz, for example.

6. Inhaler device (10) in accordance with one of the preceding claims, **characterized by** the fact that the nasal interface (30; 30a) is manufactured using a supple material.

7. Inhaler device (10) in accordance with one of the preceding claims, **characterized by** the fact that the inhalation chamber (20; 120) has a length of between 50 mm and 400 mm.

8. Inhaler device (10) in accordance with one of the preceding claims, **characterized by** the fact that the inhalation chamber (20; 120) has a volume (27; 127) of between 50 mL and 500 mL.

9. Treatment system (1) **characterized by** the fact that it includes:
• one inhaler device (10) in accordance with one of claims 1 to 8; and
• one dosing aerosol generator (40) incorporating one cartridge (50) containing a medication and being connected to the inhalation chamber (20; 120), with a manual actuation of the dosing aerosol generator (40) causing the generation of a dose of aerosol (A1) in the inhalation chamber (20; 120) for a period of less than 10 seconds and, preferably, less than 5 seconds.

10. Treatment system (1) in accordance with claim 9, **characterized by** the fact that the cartridge (50) contains a medication for the treatment of rhinosinusitis - preferably a corticoid.

11. Treatment system (1) in accordance with one of claims 9 or 10, **characterized by** the fact that the upstream opening (22) of the inhalation chamber (20) directly accommodates the dosing aerosol generator (40).

12. Treatment system (1) in accordance with one of claims 9 or 10, **characterized by** the fact that, at the upstream opening (122) of the inhalation chamber (120), the inhaler device (10) incorporates:
• one longitudinal aperture (201) accommodating the inspiration valve (70); and
• one lateral aperture (202) designed to accommodate the dosing aerosol generator (40).
